# EUROPEAN PATENT SPECIFICATION

(11) **EP 0 331 743 B1**
(45) Date of publication and mention of the grant of the patent: **18.11.1993**
(21) Application number: 88907375.5
(22) Date of filing: 22.08.1988
(51) Int. Cl.: C07K 15/06, C07K 15/04, C07K 3/20, A61K 39/00, A61K 39/395, C12N 5/00, C12N 15/00, C12P 21/00, G01N 33/53, G01N 33/577

(54) **PROTEIN DERIVED FROM LIVING BODY**
VON EINEM LEBENDEN KÖRPER ABGELEITETES PROTEIN
PROTEINE DERIVEE D'UN CORPS VIVANT

(30) Priority: 22.08.1987 JP 207403/87; 20.08.1988 JP 205690/88
(43) Date of publication of application: 13.09.1989
(73) Proprietor: MITSUI TOATSU CHEMICALS, Inc., Chiyoda-Ku Tokyo 100 (JP)
(72) Inventor: MURAMATSU, Takashi, Kagoshima-shi Kagoshima 891-01 (JP); MURAMATSU, Hisako, Kagoshima-shi Kagoshima 891-01 (JP); INOUE, Hiroshi, Kagoshima-shi Kagoshima 890 (JP); AWAYA, Akira, Yokohama-shi Kanagawa 244 (JP); FUKUI, Hideo, Mobara-shi Chiba 297 (JP); HASHIMOTO, Yoshihide, Mobara-shi Chiba 297 (JP)
(74) Representative: Schüler, Horst, Dr.
(86) International application number: JP8800833
(87) International publication number: WO8901947

(56) References cited:
- WO-A-86/04239
- JP-A-60 100 597
- JP-A-61 275 221
- JOURNAL OF BIOCHEMISTRY, vol. 96, no. 3, 1984; S. NOGUCHI et al., pp. 881-886#
- ANALYTICAL BIOCHEMISTRY, vol. 154, 1986, Academic PressInc.; R.R. LOBB et al., pp. 1-14#
- CANCER RESEARCH, vol. 43, no. 9, September 1983, C.L. ASHENDEL et al., pp. 4327-4332#

## Description

### TECHNICAL FIELD

The present invention relates to a protein (hereinafter referred to as GP68 protein) isolated, for example, from fetal mouse brains, having a molecular weight of about 68,000 and an isoelectric point of 5.4 to 5.6, which is essential for the growth and differentiation of cells in the cerebral and nervous system and the growth and development of other organs and tissues, plays an important role in promotion or regulation thereof, and is applicable to therapeutic, prophylactic and diagnostic agents for disorders due to abnormality in the cerebral and nervous system, insufficiency or excrescence in growth and development of respective organs or tissues and cancers or the like. Furthermore, the present invention relates to a process for isolating and purifying said GP68 protein, to polyclonal antibodies and monoclonal antibodies against said GP68 protein, to hybridomas capable of producing said monoclonal antibodies, and to a process for producing these antibodies.

Furthermore, the present invention relates to a pharmaceutical composition useful as therapeutic agents, prophylactic or diagnostic agents for the aforementioned various disorders.

### BACKGROUND ART

As to the research concerning proteins or the like present in tissues or cells of the cerebral and nervous system, the advancement has been slow as compared with that concerning proteins or the like present in other organs: however, much knowledge has been accumulated today.

For example, as the proteins or the like present in the tissues or cells of the cerebral and nervous system, tubulin, microtubulin-associated proteins (MAPs), neurofilament triplet and glial fibrillary acidic protein (GFA protein) are known (Structure and Function of Cell Skeleton, in Experimental Methods in Biochemistry; a second series, No. 6, Parts 1 and 2 in Vol. 1 and Part 9 in Vol. 2, Japanese Society for Biochemistry).

Investigations on various substances contained in cells of the cerebral and nervous system, especially such proteins that exert functions in differentiation of the cells of the cerebral and nervous system, as well as analyses of the functions are extremely useful in order to find proteins or the like applicable, for example, in development of new therapeutic or prophylactic agents or diagnostic means for various disorders in the cerebral and nervous system.

Consequently, the investigations on the various substances contained in the cells of the cerebral and nervous system and the analyses of the functions thereof have been highly demanded.

Under these circumstances, the present inventors revealed the presence of a period-specific protein in fetal mouse brains as one of the proteins derived from the cells of the cerebral and nervous system (Japanese Patent Laid Open No. 60-100597; Noguchi, S. et al., J. Biochem., 96, 881, 1984)

Furthermore, the presence of a protein specifically found in tumor cells of the human cerebral and nervous system has been disclosed by the present inventors in the specification of Japanese Patent Laid Open No. 61-233623.

On the other hand, the present inventors have disclosed and presence of a protein which is found in tumor cells of the cerebral and nervous system of mice and rats but not or in a minute amount, if any, in normal brain cells of mice or rats (Japanese Patent Laid Open 61-275221; Noguchi, S. et al., Cell Structure and Function, 12, 127, 1987).

### DISCLOSURE OF INVENTION

In view of the aforementioned importance of the proteins contained in the tissues and cells of the cerebral and nervous system, the inventors have been keeping searching various substances such as proteins. The search led the finding of a protein (GP68 protein) having a molecular weight of about 68,000 daltons, estimated by using two dimensional electrophoresis, and an isoelectric point of 5.4 to 5.6, which is the most prevailing protein among the proteins found in brains of mouse embryos (fetuses) and is expected to be applicable to therapeutic or prophylactic agents for disorders due to the abnormality in the cerebral and nervous system, insufficiency in the growth and development of various organs and tissues, excrescence, cancers or the like.

However, since the amount of the GP68 protein present is extremely minute and contamination of various proteins present together is unavoidable when prepared in a conventional method, it has become necessary to develop a method for obtaining a sufficient amount of highly purified GP68 protein.

Under these circumstances, the inventors of the present invention have intensively investigated to develop a more effective method of isolating and purifying the GP68 protein and found that by introducing an additional step of affinity chromatography with lectin bound as a carrier, contamination of undesired proteins is effectively removed so that a sufficient amount of highly pure GP68 protein is effectively isolated and purified. Furthermore, by the purification method based on this finding to obtain sufficient amount of the GP68 protein, production of antibodies to the GP68 protein is successfully accomplished and an efficient method of isolating and purifying the GP68 protein using the antibodies is further established, so as to complete the invention.

An object of the present invention is to provide a process of isolating and purifying a sufficient amount of highly pure GP68 protein, which is necessary in investigating properties and functions of the GP68 protein so as to confirm effectiveness of the GP68 protein as pharmaceutical preparations for human and animal use or as a diagnostic agent.

Another object of the present invention is to provide a technology necessary to develop various immunological measurements in which the GP68 protein is identified using polyclonal or monoclonal antibodies against said protein as a marker in diagnosing patients, for example, having disorders in the cerebral and nervous system, insufficiency in the growth and development of various organs or tissues, excrescence or cancers thereof, and the antibodies themselves and a process for the production thereof, or a pharmaceutical composition containing said antibodies as therapeutic and prophylactic agents.

A further object of the present invention is to provide a pharmaceutical composition containing the GP68 protein for prevention or therapy of insufficiency in the growth and development of various organs including organs in the cerebral and nervous system.

The present invention to accomplish the above-mentioned objects includes the GP68 protein having a molecular weight of about 68,000 and an isoelectric point of 5.4 to 5.6, isolated from fetal mouse brains or the like; a process for the purification of the GP68 protein; polyclonal and monoclonal antibodies against the GP68 protein; a process of producing hybridomas capable of producing said monoclonal antibodies and a process of producing these antibodies; and a useful pharmaceutical composition containing the GP68 protein or these antibodies as therapeutic, prophylactic or diagnostic agents for the above-mentioned various disorders.

### BEST MODE FOR CARRYING OUT THE INVENTION

The first process for isolating and purifying the GP68 protein according to the present invention is characterized in that a procedure using an affinity chromatography with a lectin immobilized (bound) as an affinity agent onto a carrier is introduced in a step of isolation of the GP68 protein.

Namely, a mixture of the GP68 protein and, for example, various undesired proteins is brought into contact with a carrier carrying immobilized lectin to allow the GP68 protein to adsorb to the carrier, and then the GP68 protein is selectively isolated from the carrier.

Examples of the carrier to be used in this procedure include agarose, acrylamide gel, various Toyopearl products (Tosoh Co., Ltd.) and cellulose.

Further, examples of the lectin to be immobilized onto the carrier include ricinus communis agglutinin (hereinafter referred to as RCA), wheat germ agglutinin (hereinafter referred to as WGA) and concanavalin A. Among them, RCA is most preferably used.

Immobilization of the lectin to the carrier may be carried out arbitrarily by an ordinary method suited to the kind of the carrier and lectin to be used.

An affinity chromatography with the use of the aforementioned materials may be carried out by an appropriately selected method at needs, such as a column chromatography in which a lectin-carrying carrier is filled in a column having an appropriate size and shape, or a batch method in which a lectin-carrying carrier is mixed together with a solution to be treated or a solution for elution.

With the use of the aforementioned affinity chromatography with lectin, it is possible to isolate and purify the GP68 protein by treating solutions containing the GP68 protein obtained in different steps of purification as well as extract fluids of various tissues or cells of fetal mouse brains or the like.

In the case where the GP68 protein is isolated from mouse tissues or cells, extraction is carried out by homogenizing the mouse tissues or cells for a certain period of time, for example, in 10 mM Tris-HCl buffer (pH 7.5) containing 2 % Triton X-100 (product of Rohm and Haas Co.), 0.005 % phenylmethylsulfonyl fluoride (PMSF) and 0.15 M NaCl, and then the resultant crude extract is centrifuged (for example, at 40,000 rpm) to obtain a supernatant.

Further, examples of the mouse tissues or cells to be used in the aforementioned case include those taken in the period from fertilization to one week after birth, for example, embryos (fetuses) at 11-19 days after fertilization or individual organs and tissues of mice at one week of age, such as brain, nerve tissues, intestinal tracts, smooth muscle tissues, liver, heart, kidney, lung or spleen. In the case where the isolation by extraction is to be carried out at a high concentration, fetal mouse brains or newborn mouse brains at up to about one week of age are advantageously used.

The GP68 protein adsorbed onto the lectin-carrying carrier is eluted using a solution for elution comprising constituents capable of selectively eluting the GP68 protein, so as to separate the GP68 protein from other proteins.

The solution for elution is appropriately selected according to the kind of lectin and the carrier to be used. For example, solutions of 0.01-0.2 M lactose, N-acetyl glucosamine, alpha-methylmannoside and sialic acid are applicable.

The GP68 protein in each eluted fraction is confirmed by measuring a molecular weight and an isoelectric point.

The GP68 protein in the present invention has a molecular weight of about 68,000 (68,000 ±2,000) estimated by the secondary electrophoresis as described thereinafter in Example 1, and an isoelectric point of 5.4 to 5.6.

The fraction containing the GP68 protein fractionated by the procedure described above is treated by various means, such as gel filtration, as used in various other purification procedures, so that highly purified GP68 protein can be efficiently obtained.

In the gel filtration, any method ordinarily used in protein purifications may be applicable.

The highly purified GP68 protein thus obtained has the amino acid composition and sugar composition as thereinafter described in Example 1 and the amino acid sequence of the oligopeptide in the amino terminal region is almost identical with that of mouse alpha-fetoprotein in the same region (Michael B. Gorin et al., J. Biol. Chem., 256, 1954, 1981). Furthermore, the sugar content was 12.7 % by weight.

The GP68 protein is useful as an effective constituent of a pharmaceutical composition as a medicine for prevention or treatment of insufficiency in growth and development of various organs including those in the cerebral and nervous system.

Next, the aforementioned GP68 protein can be used for immunization of animals to prepare antibodies against the GP68 protein.

More specifically, animals such as rats, rabbits, goats, horses or cattle are immunized with the GP68 protein and polyclonal antibodies against the GP68 protein are obtained in the sera of the immunized animals.

Immunization is carried out in a manner known per se, such as injection of the GP68 protein in an appropriate amount with an appropriate adjuvant into the back, foot pads, intraperitoneally or intravascularly. Antisera are collected from the animals 7-200 days after the immunization to obtain objective polyclonal antibodies.

Further, it is possible to increase the antibody titer by additional immunizations at appropriate intervals after the first immunization.

On the other hand, spleen cells of animals immunized in the aforementioned manner or of Balb/c mice are fused with myeloma cells, so as to obtain hybridomas capable of producing monoclonal antibodies against the GP68 protein.

Examples of the myeloma cell lines to be used include mouse origin myeloma cell lines such as mouse NS-1, X 63-Ag8, MPC-11 and SP-2/0 and rat 210.RCY.Ag1.2.3 cell line.

Furthermore, sera from animals at 10-80 days after the first immunization with the GP68 protein are most preferably used.

Furthermore, the fusion may be carried out in a manner which is known per se. Obtained hybridomas are cultured, for example, in an RPMI medium solution (Nissui Pharmaceutical Co. Ltd., Code 05911) containing 5-20 % fetal calf serum and HAT, then in an RPMI medium solution containing HT, and finally in a medium such as an RPMI medium for cell culture, so that desired monoclonal antibodies against the GP68 protein are excreted and accumulated in the medium. The monoclonal antibodies are thus produced.

Alternatively, the hybridomas are transplanted into the abdominal cavity of nude rats or immuno-suppressed rats to induce ascites tumor, so that the monoclonal antibodies are produced and accumulated in the ascites and thus the monoclonal antibodies are prepared.

The polyclonal and monoclonal antibodies thus obtained are useful as mentioned above as a constituent of a reagent to identify the GP68 protein as a marker in diagnosing patients having disorders, such as insufficiency in growth or development of respective organs or tissues or excrescence or cancers thereof, including disorders in the cerebral and nervous system, using various immunological measurements. Further, they are useful for the development of therapeutic and prophylactic agents containing these antibodies. For example, it is revealed when cancer tissues of various cancer patients are stained by a fluorescence staining method using polyclonal or monoclonal antibodies against the GP68 protein, tissues and cells are stained only at the site of cancer tissues, by the antibodies of the present invention. Thus, it is evident that anti-GP68 protein antibodies are useful as a diagnostic agent.

Furthermore, when gel precipitin reactions of the GP68 protein with an anti-GP68 protein polyclonal rabbit (rat) antiserum, and with an anti-mouse alpha-fetoprotein polyclonal rabbit antiserum were independently carried out using the Ouchterlony method, both of the antisera showed the same precipitin reactions against the GP68 protein, which suggested again that the GP68 protein and mouse alpha-fetoprotein were considerably homologous.

On the other hand, in the gel precipitin reaction similarly carried out according to the Ouchterlony method, unlike the reaction with the rabbit antiserum against the GP68 protein, a rabbit antiserum against human placenta alpha-fetoprotein (product of Hoechst) did not exhibit precipitin reaction with the GP68 protein. In fact, when the tissue antigen from a human cancer patient was stained using the Vectastain ABC method or a fluorescent antibody method, there appeared distinct difference in the mode of reaction between the two sera (Tables 1A and 1B). Furthermore, it was revealed that the anti-GP68 protein antiserum had a broader spectrum and reacted more intensively than the anti-human alpha-fetoprotein antiserum.

Furthermore, with the use of an affinity chromatography carrier constructed using antibodies thus obtained, the second affinity chromatography according to the present invention is carried out, so as to isolate and purify the GP68 protein.

Examples of the carrier to be used include agarose, Sephadex products, cellulose products such as BrCN-activated Sepharose 4B, Afigel 10 (Bio-Rad Laboratories) and various Toyopearl products. Binding to the carrier is carried out by dissolving antibodies in an appropriate buffer solution or the like and mixing them with the carrier at 4°C for several hours to overnight.

In the case where the affinity chromatography using antibodies against the GP68 protein is used, the elution of adsorbed GP68 protein may be carried out using an acid buffer solution, an alkaline buffer or a neutral buffer solution containing a high concentration of salt.

A pharmaceutical composition comprising the GP68 protein or anti-GP68 protein antibodies as an effective component is prepared by mixing with various appropriate additives. Examples of said additives include non-ionic surfactants such as polysorbates, e.g. Tween 80, albumin, gelatin, dextran, polyoxyethylene-solidified castor oil, fatty acid alcohol esters, polyglycol ether, phosphate-buffered saline, various amino acids, sugars such as dextrose, mannitol, glucose, xylitol, lactose, sucrose, galactose, fructose, maltose, saccharose and sorbitol. They can be used alone or in combination.

A pharmaceutical composition of the present invention is used in such a form as tablet, capsule, powder, granule, troche, package wrapped in cachet, elixir, emulsion, solution, syrup, suspension, aerosol, ointment, aseptic syringe, formed poultice, tape, soft or hard gelatin capsule, suppository or asceptically packaged powder.

Furthermore, a pharmaceutical composition of the present invention may contain, as a pharmaceutically acceptable additives for carriers or the like, besides the aforementioned examples, various substances which respectively function as a filing agent, a binding agent, a lubricating agent, a humectant agent, a decaying agent, an emulsifier, a suspending agent, a diluent, a sweetening or a flavoring agent.

Examples of the additives include, corn starch, crystallized cellulose, arabian gum, calcium phosphate, alginate, calcium silicate, fine crystal cellulose, polyvinyl pyrrolidone, tragacanth gum, gelatin, syrup, methylcellulose, carboxymethylcellulose, methyl-hydroxybenzoate esters, propyl-hydroxybenzoate esters, talc, magnesium stearate, inactive polymers, water and mineral oils.

Further, a pharmaceutical composition of the present invention may be prepared such that an active ingredient be released at a desired rate after administration.

In the case where a composition of the present invention is orally administered, for example, the GP68 protein or anti-GP68 protein antibody is mixed with the aforementioned carrier or the like to prepare into a form of tablet or capsule.

In the case where a composition of the present invention is administered parenterally, i.e. by intravenous drip or injection, or intramuscular injection, for example, an effective amount of the GP68 protein or anti-GP68 protein antibodies may be dissolved in a solution such as a glucose aqueous solution, isotonic saline, sterile water or the like, so as to bottle up into vials or ampoules.

Further, when used in vialed or ampullate forms, the GP68 protein or anti-the GP68 protein antibodies may be lyophilized in vials or ampoules.

The concentration of the GP68 protein or anti-the GP68 protein antibodies in a pharmaceutical composition of the present invention may be appropriately selected. For example, an amount from 0.1 microgram to 50 mg per unit dose may be used.

The present invention is explained in detail by Examples and Experimental examples which follow. However, these examples are intended to illustrate the invention and not to limit the scope of the invention.

### Example 1

A hundred pieces of fetal ICR mouse brains (at 13 days after fertilization) were homogenized in 100 ml of 10 mM Tris-HCl buffer (pH 7.6) containing 2 % Triton X-100, 0.005 % PMSF and 0.15 M sodium chloride and subjected to extraction procedure at 4°C for 30 minutes. The obtained crude extract was centrifuged at high speed at 120,000 x g for 1 hour and the resultant supernatant was applied to an RCA agarose affinity chromatography column (4 ml) prepared as hereinafter described. The column was washed with 40 ml of the aforementioned buffer solution.

### Application to column:

RCA agarose (EY Laboratories) was filled into a 4 ml-volume column and then equilibrated with 10 mM Tris-HCl buffer (pH 7.5) containing 1 % Triton X-100 and 0.005 % PMSF.

Subsequently, elution was carried out with 20 ml of 10 mM Tris-HCl (pH 7.5) containing 1 % Triton X-100 and 0.005 % PMSF supplemented with 0.1 M lactose, so as to obtain an eluate solution.

A portion of the eluate was thoroughly dialyzed to obtain a purified protein solution and lyophilized to obtain a protein preparation.

Further, the protein yield of the protein preparation was 2.0 mg per 100 pieces of the fetal mouse brains.

Furthermore, the protein content was determined by the Lowry method using bovine serum albumin (Sigma) as a reference standard. Further, the protein content determinations in the procedures described below were similarly carried out.

On the other hand, separately, a remaining portion of the aforementioned eluate solution was precipitated with an addition of ethanol at a final concentration of 80 to 90 % to obtain an ethanol-precipitated protein, so as to obtain a protein preparation.

A portion of the obtained protein preparation was subjected to the two dimensional electrophoresis as described below to determine the molecular weight and the isoelectric point. As a result, a protein having a molecular weight of approximately 68,000 and an isoelectric point of 5.4 to 5.6 was exclusively detected.

Further, reference protein standards used in the molecular weight determination were thyroglobulin (M. W., 330,000), lactoferin (M. W., 88,000), bovine serum albumin (M. W., 67,000), egg white albumin (M. W., 43,000) and aldolase (M. W., 34,000), all of them being products of Sigma.

### Conditions for two dimensional electrophoresis:

### a) One dimensional electrophoresis

A medium to be used for the electrophoresis comprised 2.76 g urea, 0.67 ml of 30 % acrylamide, 1 ml of 10 % NP-40, 25 ml of ampholine (pH 3.5 to 10), 10 microliters of 10 % ammonium persulfate, 0.14 ml of 5 % tetramethylethylenediamine (TEMED, Nakarai Chemicals, Ltd.) and 0.91 ml of water. A sample was subjected to electrophoresis, using 10 mM H₃PO₄ for a cathode solution and 20 mM NaOH as an anode solution, at 400 V for 13 hours and then at 800 V for 1 hour.

### b) Two dimensional electrophoresis

After the one dimensional electrophoresis, the gel was equilibrated with sodium dodecyl sulfate sample buffer [10 % glycerin, 5 % beta-mercaptoethanol, 23 % SDS, 62.5 mM Tris-HCl buffer (pH 6.8)] and subjected to the two dimensional electrophoresis on 7.5 % acrylamide slab gel (25 mM Tris, 192 mM glycine, 0.1 % SDS) at 120 V for 4 hours.

After the electrophoresis, the gel was stained for 1 hour in a solution containing 0.05 % Coomassie blue, 10 % methyl alcohol and 10 % acetic acid, treated with 10 % methyl alcohol and then with 10 % acetic acid and then the obtained spots were respectively measured.

Then, the sugar content of the previously obtained lyophilized preparations (1 mg of protein) of the GP68 protein was determined using gas liquid chromatography after methylation analysis by the method of Bhatti et al.

On the other hand, the sialic acid content of the lyophilized preparation was determined by thiobarbituric acid reaction using N-acetylneuraminic acid as a standard, after hydrolysis reaction with 0.05 M H₂SO₄ at 80°C for 30 minutes.

Furthermore, a portion of the lyophilized preparation (1 mg of protein) was hydrolyzed with 4 M HCl at 100°C for 4 hours and the obtained hydrolysate was analyzed for hexosamine using Hitachi amino acid analyzer Model 853. Another portion of the lyophilized preparation (98 micrograms of protein) was similarly hydrolyzed with 6 M HCl at 105°C for 24 hours and analyzed for amino acid composition using the same analyzer.

Results are as follows:

| Sugar composition (molar ratio): | |
|---|---|
| Galactose | 1 |
| Mannose | 1.42 |
| Fructose | 0.32 |
| Glucose | 0.07 |
| Glucosamine | 1.06 |
| Galactosamine | 0.10 |
| Sialic acid | 0.18 |

| Amino acid composition (molar ratio): | |
|---|---|
| Aspartic acid | 43.2 |
| Threonine | 26.1 |
| Serine | 35.8 |
| Glutamic acid | 58.4 |
| Glycine | 30.2 |
| Alanine | 30.6 |
| Cysteine | 6.9 |
| Valine | 23.3 |
| Methionine | 5.2 |
| Isoleucine | 9.5 |
| Leucine | 48.4 |
| Tyrosine | 8.6 |
| Phenylalanine | 17.4 |
| Lysine | 26.3 |
| Ammonium | 90.1 |
| Histidine | 10.6 |
| Arginine | 18.2 |
| Proline | 26.5 |

Furthermore, the aforementioned lyophilized preparation of the GP68 protein (105 micrograms) was dissolved in 25 microliters of water and a 17 microliters portion (71.4 micrograms) was applied to a peptide sequencer (Applied Science, Model 477A) to analyze the amino acid sequence of the N-terminal. As a result, the following amino acid sequence was revealed:
Amino acid sequence of the N-terminal of the GP68 protein: 
Leu-His-Glu-Asn-Glu-Phe-Gly-Ile-Ala-Ser-Thr-
Leu-Asp-Ser-X-Gln-Y-Lys-Thr-Glu-Lys-
(X and Y were not confirmed)
Further, for reference, the amino acid sequence of the N-terminal of mouse alpha-fetoprotein is as follows; Michael B., Gorin et al., J. Biol. Chem. 256, 1954- (1981): Amino acid sequence of the N terminal of mouse alpha-fetoprotein protein: 
Leu-His-Glu-Asn-Glu-Phe-Gly-Ile-Ala-Ser-Thr-
Leu-Asp-Ser-Ser-Gln-Cys-Val-Thr-Glu-Lys-
Furthermore, precipitin reactions in gel were carried out, according to the Ouchterlony method, regarding the GP68 protein (the aforementioned lyophilized preparation), with an anti-mouse GP68 protein polyclonal rabbit antiserum (prepared in Example 2), and with anti-mouse alpha-fetoprotein polyclonal rabbit antiserum (product of Hoechst), independently. Both of the antisera showed the similar precipitin reactions against the GP68 protein.

### Example 2

Rabbits (New Zealand White) were immunized, at foot pads, by injection of 100 micrograms of a mixture of the lyophilized preparation of the GP68 protein and the ethanol precipitated preparation (both obtained in Example 1) at a ratio of 1:1 dissolved in complete Freund adjuvant (Nakarai Chemicals, Ltd.)

Ten days after completion of the immunization, all the blood was collected and serum was prepared. The obtained serum was run through an Afigel 10 (Bio Rad Laboratories Co.) column and the resulting eluate was furthermore run through a column filled with Afigel 10 which had been allowed to contact with sodium bicarbonate buffer (pH 8.0) containing 1 mg/ml of the GP68 protein and then antibodies were adsorbed on the column. Subsequently, the adsorbed antibodies were purified by the elution with a 3 M potassium thiocyanate solution. The purification procedures were repeated several times to prepare a sufficient amount of antibody solution.

The eluted fraction containing the antibodies was thoroughly dialyzed. It was confirmed by immunoblotting analysis at a protein concentration of 20 micrograms/ml that polyclonal antibodies against the GP68 protein were contained in the fraction.

### Example 3

Goats were immunized by intracutaneous and subcutaneous injection of 500 micrograms of a mixture of the lyophilized preparation of the GP68 protein and the ethanol precipitated preparation (both obtained in Example 1) at a ratio of 1:1 dissolved in complete Freund adjuvant at 2 weeks intervals totally four times. The blood was collected at intervals.

Whole blood was collected 5 months after the immunization, when the titer was sufficiently increased. The obtained serum was purified using columns in the same manner as described in Example 2, so as to accumulate polyclonal antibodies against the GP68 protein. An analysis of a portion of the antibodies revealed that the antibodies are specific to the GP68 protein.

### Example 4

Polyclonal antibodies were collected in the same manner as in Example 3, except that the animals to be immunized were horses.

### Example 5

Polyclonal antibodies were collected in the same manner as in Example 3, except that the animals to be immunized were cattle.

### Example 6

Wistar rats (5 to 6 weeks of age) were immunized by injection of a mixture of the lyophilized preparation of the GP68 protein and the ethanol precipitated preparation (both obtained in Example 1) at a ratio of 1:1 in the amounts shown below at 2 weeks intervals.

| | |
|---|---|
| 1st (subcutaneously) | 50 micrograms |
| 2nd (subcutaneously) | 50 micrograms |
| 3rd (intraperitoneally) | 100 micrograms |
| 4th (intraperitoneally) | 100 micrograms |

Spleen cells (1 x 10⁸) collected three days after the final immunization were subjected to fusion with 2 x 10⁷ cells of the mouse NS-1 cell line used as myeloma cells in the presence of polyethylene glycol 400 according to the method of Koehler et al.

Polyethylene glycol was removed and resultant hybridomas were suspended in 200 ml of HAT medium [RPMI 1640 medium containing HAT and 10 % fetal calf serum (FCS)]. The suspension was applied to a 96-well plate (Falcon) to dispense 1 x 10⁵ cells per well.

Then, incubation was continued at 37°C for 1 week to 10 days.

Every 3 days after 10-day incubation, half a portion of supernatant of the cell culture of individual wells was subjected to the enzyme linked immuno sorbent assay (ELISA) according to the Vectastain ABC method using the avidine-biotinized peroxidase complex method (Funakoshi Pharmaceutical Co., Ltd.) so as to detect positive wells. A fresh medium solution was added to the wells and incubation was further continued. Furthermore, desired clones were obtained by cloning the cells in positive wells using a limiting dilution method.

Further, the assay plates 2095 (Falcon) used in the ELISA was brought into contact in an ordinary manner with phosphate buffered saline (PBS) in which the ethanol-precipitated GP68 protein obtained in Example 1 was dissolved so that 100 ng each of the GP68 protein was adsorbed to the individual wells.

As a result, a total of 8 positive clones were obtained.

Among these clones, two clones (EBR 3, EBR 7) were independently used to obtain EBR 3-1 monoclonal antibodies and EBR7-1 monoclonal antibodies as follows:

Further, the clones EBR 3 and EBR 7 were deposited according to the Budapest Treaty at the Fermentation Research Institute, Agency of Industrial Science and Technology (1-3 Higashi 1-chome, Tsukuba-shi, Ibaraki Prefecture, Japan 305).

The deposition was made on August 18, 1988 under the deposition numbers FERM BP-2002 for clone EBR 3 and FERM BP-2003 for clone EBR 7.

### Example 7

Monoclonal antibodies against the GP68 protein were prepared using the hybridomas (clones EBR 3 and EBR 7) obtained in Example 6.

Firstly, clones EBR 3 and EBR 7 were incubated independently in RPMI 1640 medium in 10-cm petri dishes.

Precipitates were obtained from the supernatants of respective cultures by precipitation with 50 % ammonium sulfate. The precipitates were dissolved in 1 to 2 ml of PBS and dialyzed against PBS for 3 days. After the dialysis, the solutions were independently applied to Sephadex G-250 columns to obtain purified EBR 3-1 monoclonal antibodies and purified EBR 7-1 monoclonal antibodies.

Furthermore, 8-week-old nude rats, which had been intraperitoneally injected with 0.5 ml/rat of 2,6,10,14-tetramethylpentadecan and grown for 1 to 2 week (pristane treatment), were intraperitoneally injected independently with 1 x 10⁷/ rat of clone EBR 3 or clone EBR 7, both obtained in Example 6. Ascites accumulated (50 ml/rat) was removed from the rats at 10 to 21 days after the injection and centrifuged to remove solid substances.

The obtained supernatants were salted out with 50 % ammonium sulfate then with 40 % ammonium sulfate. The resultant supernatants were dialyzed against PBS (pH7.2) for 3 days.

The obtained crude monoclonal antibodies were applied to a Sephadex G-200 column. Elution was curried out with PBS so as to recover solutions containing two kinds of purified monoclonal antibodies (EBR 3-2 monoclonal antibody and EBR 7-2 monoclonal antibody) independently.

### Example 8

Purified monoclonal antibodies (EBR 3-3 monoclonal antibody and EBR 7-3 monoclonal antibody) were obtainable by repeating the procedures similar to those in Example 6 and Example 7, except that Balb/c mice were used in the place of Wistar rats for hybridoma preparation.

### Example 9

According to an ordinary method, a column with immobilized polyclonal antibodies was prepared by bringing Afigel 10 into contact with the buffer solution of polyclonal antibodies obtained in Example 2, at 4°C for about 24 hours.

The fetal mouse brain extract obtained in Example 1 was treated with this column. The GP68 protein was thus efficiently isolated and purified.

### Example 10

A fetal mouse brain extract was treated in the same manner as described in Example 9, except that the purified monoclonal antibodies (EBR 3-1 monoclonal antibodies and EBR 7-1 monoclonal antibodies) were used. The GP68 protein was thus efficiently isolated and purified.

### Example 11

The purified protein solution obtained in Example 1 (obtained after dialysis of the eluate from the RCA agarose column) was dispensed into vials (100 micrograms of the GP68 protein in a vial) and lyophilized. A saline solution of 1.0 ml supplemented with 0.001 to 0.1 % of Tween 80 was then added to each vials and the vials were plugged. A pharmaceutical preparation containing the GP68 protein was thus obtained.

### Example 12

A pharmaceutical preparation containing the GP68 protein was obtained in the same manner as described in Example 11, except that a saline solution supplemented with 0.001 to 10 % albumin was used in the place of Tween 80.

### Example 13

A pharmaceutical preparation containing the GP68 protein was obtained in the same manner as described in Example 11, except that a saline solution supplemented with 0.01 to 5 % glucose was used in the place of Tween 80.

### Example 14

A pharmaceutical preparation containing the GP68 protein was obtained in the same manner as described in Example 11, except that a saline solution supplemented with 0.01 to 5 % mannitol was used in the place of Tween 80.

### Examples 15a to 15d

A pharmaceutical preparation containing the GP68 protein was obtained in the same manner as described in Examples 11 to 14, except that the amount of the GP68 protein to be lyophilized in a vial was 1 mg.

### Example 16

Solutions independently containing the two purified monoclonal antibodies obtained in Example 7 were dispensed into vials (100 micrograms of anti-GP68 protein monoclonal antibodies in a vial) and lyophilized. A saline solution of 1.0 ml supplemented with 0.001 to 0.1 % of Tween 80 was then added to respective vials and the vials were plugged. A pharmaceutical preparation containing anti-GP68 protein monoclonal antibodies was thus obtained.

### Example 17

A pharmaceutical preparation containing anti-GP68 protein monoclonal antibodies was obtained in the same manner as described in Example 16, except that a saline solution supplemented with 0.001 to 10 % albumin was used in the place of Tween 80.

### Example 18

A pharmaceutical preparation containing anti-GP68 protein monoclonal antibodies was obtained in the same manner as described in Example 16, except that a saline solution supplemented with 0.01 to 5 % mannitol was used in the place of Tween 80.

### Example 19

A pharmaceutical preparation containing anti-GP68 protein monoclonal antibodies was obtained in the same manner as described in Example 16, except that a saline solution supplemented with 0.01 to 5 % glucose was used in the place of Tween 80.

### Experimental example 1

Using the polyclonal antibodies obtained in Example 2, extracts, by Triton-X, of the mouse embryos/fetuses and various organs of 1- to 30-day-old mice were analyzed by the western blotting method as follows:
Firstly, the mouse embryos (at 13 days after fertilization) and the various mouse organs (e.g. brain, ganglion, heart, lung, liver, gastrointestinal tracts, spleen) were treated to obtain extracts as described below.

Samples were homogenized in 0.01 M Tris-HCl buffer containing 2 % Triton X-100 and 50 micrograms/ml PMSF and subjected to extraction on ice for 30 minutes. The resultant extracts were centrifuged at 40,000 rpm for 1 hour and the obtained supernatants were subjected to ethanol precipitation. The amount of proteins contained in the resultant precipitates were determined by the Lowry method.

Then, according to the Laemmli method (Nature 227, 680-685, 1970), each of the precipitates was dissolved to give a final concentration of 4 mg/ml in an SDS solubilizing solution [9.5 M urea, 2 % NP-40 (Nakarai Chemicals, Ltd.), 2 % ampholine (Pharmacia), 5 % mercaptoethanol, 0.25 % SDS]. The solutions were independently applied on SDS-polyacrylamide gel electrophoresis (gel concentration: 10 %; 10 to 15 microliters/ lane, i.e. 40 to 60 micrograms of protein/lane).

After the electrophoresis, according to the method of Towbin et al (Towbin et al, Proc. Natl. Acad. Sci. 76, 4350-4354, 1979), the protein fractions fractionated in the aforementioned gel electrophoresis were subjected, maintaining their positions, to blotting on a nitrocellulose membrane (Schleicher & Schuell or Bio-Rad Laboratories) at 1 ampere for 2 hours. After the blotting, the nitrocellulose membrane was brought into contact with PBS containing 3 % bovine serum albumin (BSA) for 1 hour.

Then, the nitrocellulose membrane was washed with PBS containing 1 % Triton X-100, and was treated with antibodies to GP68 protein (100- to 500-fold-diluted rat or rabbit antiserum) at room temperature for 2 to 3 hours.

Furthermore, the nitrocellulose membrane was washed several times with PBS containing 1 % Triton X-100 for 1 hour each time and then treated for 1 hour with a solution containing to HRP-conjugated anti-rat IgG or anti-rabbit IgG mouse IgG antibodies (100-fold dilution). HRP (horse radish peroxidase) was a product of Cappel.

Subsequently, the membrane was washed 5 to 6 times with PBS containing 1 % Triton X-100 for 1 hour each time and then treated with 0.05 % 4-chloro-1-naphtol and H₂O₂ in 40 mM Tris-HCl buffer (pH 7.6), so that grayish-brown spots were visually observed.

As a result, in all the samples of the extracts from the organs of mouse fetuses (embryos), stained spots were identified.

For example, stained spots were observed in all the extracts from the head, abdomen and tail portions of mouse embryos at 13, 15 and 17 days after fertilization.

On the other hand, stained spots were also observed in the extracts from various organs and cells of the 1- to 7-day-old mice.

For example, stained spots were observed in all the extracts from the head, abdomen and tail portions of the mice immediately after birth. Furthermore, stained spots were observed in all the samples of extracts from the brain, liver, kidney, lung, heart, skin, spleen, muscle portions of the mice at 7 days of age. Among them, the spots in the samples from the liver and heart were intensively stained and the spot in the extract from the brain was diffused and weakly stained.

On the contrary, none of the spot was observed in the extracts from various organs and cells of the mice at over 1 week of age.

For example, none of the spot was observed in the extracts from the brain, liver, kidney, lung, heart, skin, spleen and muscle portions of the mice at 14 days of age and 21 days of age as well as from the brain, liver, kidney, lung, heart, skin, spleen, muscle, uterus and spinal cord portions of adult mice.

Accordingly, the GP68 protein is regarded as a protein having a biological activity which appears specifically in the period of embryos and shortly after birth, when growth of differentiation is extremely vigorous.

### Experimental example 2

Embryos of mice at 15 days after fertilization were rapidly chilled in dry ice/acetone and then cut into pieces using a cryostat. To the pieces added 10-fold-diluted solutions containing EBR 3-1 monoclonal and EBR 7-1 monoclonal antibodies, respectively. The resultant mixtures were allowed to react with HRP-conjugated anti-rat IgG mouse IgG antibody for 1 to 2 hours. After washing, stained organs were observed using an optical microscope. Furthermore, observation was made using the ABC method described in example 6.

As a result, it was observed that the nerve and smooth muscle were stained in the reaction with EBR 3-1 monoclonal antibodies and the brain, ganglion and liver were stained in the reaction with EBR 7-1 monoclonal antibodies.

### Experimental example 3

Brain cells of mouse embryos were taken at daily intervals to make tissue cultures. To the respective tissue cultures added independently EBR 3-1 monoclonal antibodies or EBR 7-1 monoclonal antibodies obtained in Example 7, so as to observe the brain cell activity.

### Experiment example 4

Cancer tissues obtained from various cancer patients were stained according to the aforementioned ABC method using the anti-mouse GP68 protein rabbit antiserum or the anti-human alpha-fetoprotein rabbit antiserum (both prepared in Example 2), independently.

The obtained results are shown in Tables 1A and 1B. In Tables, degrees of the staining are designated as follows: "-", not stained, "+-", weakly and diffusedly stained; "+", moderately stained, "++", distinctly stained but less strongly than those designated with "+++"; and "+++", distinctly stained.

On the other hand, none of mouse fibroblast cells, any cells of healthy humans or cells of normal tissues other than cancer tissues of cancer patients was stained when similar staining procedures were performed, which implicated that the anti-mouse GP68 protein rabbit antiserum specifically reacts with cancer tissues.

Furthermore, as evident from Tables 1A and 1B, the rabbit antiserum to the GP68 protein stained tissues of cancer patients, which the rabbit antiserum to human alpha-fetoprotein did not stain, and distinctly and more intensively stained than the latter, namely similarly or more distinctly stained. Thus, the antiserum to the GP68 protein was considered to be highly useful as a constituent of a diagnostic agent for cancers.

**Table 1A**

| Case No. | Diagnosis | Site | Staining with antiserum to | |
|---|---|---|---|---|
| | | | GP68 protein | human-alpha fetoprotein |
| 1 | 13-week-old human embryo | | +++ | +++ |
| 2 | Astrocytoma | Brain | - | - |
| 3 | do. | do. | +- | +- |
| 4 | do. | do. | - | - |
| 5 | do. | do. | - | - |
| 6 | do. | do. | +- | +- |
| 7 | Craniopharyngioma | do. | +- | +- |
| 8 | Glioblastoma multiform | do. | - | - |
| 9 | Glioma | do. | +- | +- |
| 10 | Teratoblastom | do. | +- | - |
| 11 | Medullblastoma | do. | +- | +- |
| 12 | do. | do. | +- | +- |
| 13 | Meningioma | do. | - | - |
| 14 | do. | do. | - | - |
| 15 | do. | do. | +- | +- |
| 16 | do. | do. | +- | +- |
| 17 | do. | do. | +- | +- |
| 18 | Premordial ectoblastoma | do. | - | - |
| 19 | Liver, almost normal | Liver | +- | +- |
| 20 | Cholangioma | do. | ++ | +- |
| 21 | do. | do. | +++ | +- |
| 22 | Cancer of the liver (Hepatom) | do. | - | - |
| 23 | do. | do. | ++ | + |
| 24 | do. | do. | + | +- |
| 25 | do. | do. | + | + |
| 26 | do. | do. | +- | +- |
| 27 | do. | do. | + | +- |
| 28 | do. | do. | + | +- |
| 29 | do. | do. | + | + |
| 30 | do. | do. | - | - |
| 31 | do. | do. | ++ | + |
| 32 | Liver cirrhosis | do. | + | +- |
| 33 | do. | do. | +- | +- |
| 34 | do. | do. | + | - |
| 35 | do. | do. | +- | - |

**Table 1B**

| Case No. | Diagnosis | Site | Staining with antiserum to | |
|---|---|---|---|---|
| | | | GP68 protein | human-alpha fetoprotein |
| 36 | Adenocarcinoma | Lung | ++ | - |
| 37 | Adenocarcinoma of the lung | do. | + | - |
| 38 | do. | do. | ++ | - |
| 39 | do. | do. | +- | - |
| 40 | do. | do. | +- | - |
| 41 | Ovarian cystome | Ovary | ++ | + |
| 42 | do. | do. | ++ | ++ |
| 43 | Adenocarcinoma | Stomach | ++ | - |
| 44 | do. | do. | ++ | ++ |
| 45 | do. | do. | + | + |
| 46 | Differentiation-type adenocarcinoma | do. | ++ | +- |
| 47 | Embryonal carcinoma | Testicle | + | - |
| 48 | do. | do. | + | - |
| 49 | do. | do. | ++ | + |
| 50 | do. | do. | ++ | + |
| 51 | do. | do. | ++ | +- |
| 52 | do. | do. | ++ | + |
| 53 | Seminoma | do. | + | - |
| 54 | do. | do. | + | - |
| 55 | do. | do. | ++ | - |
| 56 | do. | do. | + | +- |
| 57 | do. | do. | +- | +- |
| 58 | do. | do. | ++ | + |
| 59 | do. | do. | ++ | - |
| 60 | do. | do. | + | + |
| 61 | do. | do. | ++ | - |
| 62 | do. | do. | + | - |
| 63 | do. | do. | + | - |
| 64 | do. | do. | ++ | +- |
| 65 | do. | do. | ++ | - |
| 66 | do. | do. | + | - |
| 67 | do. | do. | + | - |
| 68 | do. | do. | + | - |
| 69 | Deformed differentiation-type adenocarcinoma | Uterus | ++ | - |

### INDUSTRIAL APPLICABILITY

The present invention provides the GP68 protein, in an isolated form, which appears period-specifically in the course of development and differentiation of the cerebral and nervous system and may act an important role in the course of the development and differentiation thereof.

Furthermore, a process for purification according to the present invention now makes it possible to obtain a sufficient amount of highly purified GP68 protein.

Furthermore, by using antibodies against the GP68 protein according to the present invention, various investigations with the use of the antibodies are to be easily promoted.

Incidences of new functions are expected by administering the GP68 protein of the present invention to adults, since the GP68 protein is present only in a specific period, i.e. from fertilization to one week after birth and not produced in adults.

Furthermore, by the use of the GP68 protein according to the present invention, effects are expected in promoting the development of organs, tissues, cells and membranes of various parts of the body, and in reforming genetic or organic abnormalities in development and growth of various organs and tissues and in regenerating and restoring the cerebral and nervous system.

Preparations of m-RNA and c-DNA coding for the GP68 protein are facilitated according to the present invention in which polyclonal and monoclonal antibodies against the GP68 protein are prepared, so that the way may be opened to produce the GP68 protein according to genetic recombinant technique, for example, using cells of E. coli, Bacillus subtilis, yeast and various animals.

Furthermore, the GP68 protein or the active center peptide thereof and the antibodies specific thereto may be expected to be useful as therapeutic or prophylactic agents for various disorders in the cerebral and nervous system; various agents for promoting growth and development; various therapeutic agents for growth insufficiency; medicines for excrescence, carcinomas or cancers; medicines for animal use; or markers for disorders in the cerebral and nervous system, insufficiency in growth and development, excrescence or cancers in various organs and tissues; and further as a diagnostic agent.

### REFERENCE TO THE DEPOSITION OF MICROORGANISMS

### 1. Clone EBR 3

### Deposition Institute:

Name: Fermentation Research Institute Agency of Industrial Science and Technology, Ministry of Industry and Trade
Address: 1-3 Higashi 1-chome, Tsukuba-shi, Ibaraki Prefecture 305, Japan
Date of Deposition: August 18, 1988
Deposition Number: FERM BP-2002
(Deposited under the Budapest Treaty)

### 2. Clone EBR 7

### Deposition Institute:

Name: Fermentation Research Institute Agency of Industrial Science and Technology, Ministry of Industry and Trade
Address: 1-3 Higashi 1-chome, Tsukuba-shi, Ibaraki Prefecture 305, Japan
Date of Deposition: August 18, 1988
Deposition Number: FERM BP-2003
(Deposited under the Budapest Treaty) .pa

## Claims

1. A protein which is specifically found in biological materials from embryos/fetuses or newborns at up to one week of age and has a molecular weight of about 68,000 and an isoelectric point of 5.4 to 5.6. having
a) a N-terminal amino acid sequence of 
Leu-His-Glu-
Asn-Glu-Phe-Gly-Ile-Ala-Ser-Thr-Leu-Asp-Ser-;
 and
b) the following sugar and amino acid compositions:
| Sugar composition (molar ratio): | |
|---|---|
| Galactose | about 1 |
| Mannose | about 1.42 |
| Fructose | about 0.32 |
| Glucose | about 0.07 |
| Glucosamine | about 1.06 |
| Galactosamine | about 0.10 |
| Sialic acid | about 0.18 |
| Amino acid composition (molar ratio): | |
|---|---|
| Aspartic acid | about 43.2 |
| Threonine | about 26.1 |
| Serine | about 35.8 |
| Glutamic acid | about 58.4 |
| Glycine | about 30.2 |
| Alanine | about 30.6 |
| Cysteine | about 6.9 |
| Valine | about 23.3 |
| Methionine | about 5.2 |
| Isoleucine | about 9.5 |
| Leucine | about 48.4 |
| Tyrosine | about 8.6 |
| Phenylalanine | about 17.4 |
| Lysine | about 26.3 |
| Ammonium | about 90.1 |
| Histidine | about 10.6 |
| Arginine | about 18.2 |
| Proline | about 26.5 |

2. A protein as set forth in claim 1, wherein said biological materials are mouse origin.

3. A protein set forth in claim 1, wherein said protein is isolated from an extract fluid of tissues or cells of mice by an affinity chromatography using a lectin as an affinity agent.

4. A process for producing a protein as set forth in claim 1, comprising the steps of bringing an extract fluid of tissues or cells of embryos/fetuses or newborns at up to one week of age into contact with a lectin-carrying carrier and selectively eluting and isolating the protein having a molecular weight of about 68,000 daltons and an isoelectric point of 5.4 to 5.6 from constituents absorbed onto the carrier.

5. A process for producing a protein as set forth in claim 4, characterized in that said extract is from mouse tissues or cells of embryos/fetuses or newborn mice at up to one week of age.

6. Polyclonal antibodies against a protein as set forth in claim 1 which has a molecular weight of about 68,000 and an isoelectric point of 5.4 to 5.6 and is specifically found in biological materials from embryos/fetuses or newborns at up to one week of age and obtained by immunization of animals with said protein.

7. Polyclonal antibodies as set forth in claim 6, wherein said biological materials are mouse origin.

8. Hybridomas capable of producing monoclonal antibodies specific to a protein as set forth in claim 1 which is specifically found in biological materials from embryos/fetuses or newborns at up to one week of age and has a molecular weight of about 68,000 and an isoelectric point of 5.4 to 5.6, said hybridomas being obtained by fusion of myeloma cells with spleen cells from animals immunized with said protein.

9. Hybridomas as set forth in claim 8 wherein biological materials are mouse origin.

10. Monoclonal antibodies specific to a protein as set forth in claim 1 which has a molecular weight of about 68,000 and an isoelectric point of 5.4 to 5.6 and is specifically found in biological materials from embryos/fetuses or newborns at up to one week of age.

11. Monoclonal antibodies as set forth in claim 10, which are produced by hybridomas obtained by the fusion of myeloma cells with spleen cells of animals immunized with a protein having a molecular weight of about 68,000 and an isoelectric point of 5.4 to 5.6 and being specifically found in biological materials from embryos/fetuses or newborns at up to one week of age.

12. Monoclonal antibodies as set forth in claim 11 wherein said biological materials are mouse origin.

13. A process for producing monoclonal antibodies against a protein as set forth in claim 1 which is specifically found in biological materials from embryos/fetuses or newborns at up to one week of age and has a molecular weight of about 68,000 and an isoelectric point of 5.4 to 5.6 said process comprising the steps of (A) fusing myeloma cells with spleen cells of animals immunized with said protein to obtain hybridomas and (B) among the hybridomas selecting those capable of producing monoclonal antibodies against said protein and producing said monoclonal antibodies using said hybridomas.

14. A process for producing monoclonal antibodies as set forth in claim 13, wherein step (B) comprises cultivating said hybridomas capable of producing said monoclonal antibodies in a tissue culture fluid, producing and accumulating said monoclonal antibodies in said culture fluid, and then isolating said monoclonal antibodies from said culture fluid.

15. A process for producing monoclonal antibodies as set forth in claim 13, wherein step (B) comprises transplanting said hybridomas capable of producing said monoclonal antibodies into the peritoneum of a nude rat or immuno-suppressed rat to induce ascites tumor, producing and accumulating said monoclonal antibodies in the ascites, and then isolating said monoclonal antibodies from said ascites.

16. A process for producing monoclonal antibodies as set forth in claim 13, 14 or 15, wherein said biological materials are mouse origin.

17. A process for isolating a protein, comprising steps of bringing a mixture containing a protein as set forth in claim 1 into contact with a carrier carrying antibodies against the protein set forth in claim 1 and selectively eluting and siolating said protein from constituents adsorbed on the carrier.

18. A process for isolating a protein as set forth in claim 17, wherein said antibodies are polyclonal antibodies as set forth in claim 6.

19. A process for isolating a protein as set forth in claim 17, wherein said antibodies are monoclonal antibodies as set forth in claim 10.

20. A pharmaceutical composition which contains a protein as set forth in claim 1.

21. A pharmaceutical composition which contains antibodies as set forth in claim 6.

22. A pharmaceutical composition which contains antibodies as set forth in claim 10.

## Patentansprüche

1. Ein Protein, das spezifisch in biologischen Materialien von Embryonen/Föten oder Neugeborenen von bis zu einer Woche ihres Lebensalters gefunden wird und ein Molekulargewicht von etwa 68.000 und einen isoelektrischen Punkt von 5,4 bis 5,6 aufweist, mit
a) einer N-endständigen Aminosäuresequenz von 
Leu-His-Glu-Asn-Glu-Phe-Gly-Ile-Ala-Ser-Thr-
Leu-Asp-Ser-;
 und
b) den folgenden Zucker- und Aminosäurezusammensetzungen:
| Zuckerzusammensetzung (molarer Anteil): | |
|---|---|
| Galactose | etwa 1 |
| Mannose | etwa 1,42 |
| Fructose | etwa 0,32 |
| Glucose | etwa 0,07 |
| Glucosamin | etwa 1,06 |
| Galactosamin | etwa 0,10 |
| Sialsäure | etwa 0,18 |
| Aminosäurezusammensetzung (molarer Anteil): | |
|---|---|
| Asparaginsäure | etwa 43,2 |
| Threonin | etwa 26,1 |
| Serin | etwa 35,8 |
| Glutaminsäure | etwa 58,4 |
| Glycin | etwa 30,2 |
| Alanin | etwa 30,6 |
| Cystein | etwa 6,9 |
| Valin | etwa 23,3 |
| Methionin | etwa 5,2 |
| Isoleucin | etwa 9,5 |
| Leucin | etwa 48,4 |
| Tyrosin | etwa 8,6 |
| Phenylalanin | etwa 17,4 |
| Lysin | etwa 26,3 |
| Ammonium | etwa 90,1 |
| Histidin | etwa 10,6 |
| Arginin | etwa 18,2 |
| Prolin | etwa 26,5 |

2. Ein Protein nach Anspruch 1, bei dem diese biologischen Materialien von Mäusen herstammen.

3. Ein Protein nach Anspruch 1, bei dem dieses Protein von einem Extraktfluid von Geweben oder Zellen von Mäusen durch eine Affinitätschromatographie unter Verwendung eines Lectins als einem Affinitätsmittel isoliert worden ist.

4. Ein Verfahren zum Herstellen eines Proteins, wie es in Anspruch 1 angegeben ist, das die Schritte umfaßt, daß ein Extraktfluid von Geweben oder Zellen von Embryonen/Föten oder Neugeborenen mit einem Lebensalter bis zu einer Woche mit einem Lectin-tragenden Träger in Kontakt gebracht wird und das Protein mit einem Molekulargewicht von etwa 68.000 Daltons und einem isoelektrischen Punkt von 5,4 bis 5,6 von auf dem Träger absorbierten Bestandteilen selektiv eluiert und isoliert wird.

5. Ein Verfahren zum Herstellen eines Proteins nach Anspruch 4, dadurch gekennzeichnet, daß der Extrakt von Mäusegeweben oder -zellen von Embryonen/Föten oder neugeborenen Mäusen mit einem Lebensalter bis zu einer Woche herstammt.

6. Polyclonale Antikörper gegen ein Protein nach Anspruch 1, das ein Molekulargewicht von etwa 68.000 und einen isoelektrischen Punkt von 5,4 bis 5,6 aufweist und spezifisch in biologischen Materialien von Embryonen/Föten oder Neugeborenen mit einem Lebensalter von bis zu einer Woche gefunden wird und durch Immunisierung von Tieren mit diesem Protein erhalten wird.

7. Polyclonale Antikörper nach Anspruch 6, bei denen die biologischen Materialien von Mäusen herstammen.

8. Hybridomas, die in der Lage sind, monoclonale Antikörper zu produzieren, die für ein Protein spezifisch sind, wie es in Anspruch 1 angegeben ist, das spezifisch in biologischen Materialien von Embryonen/Föten oder Neugeborenen mit einem Lebensalter von bis zu einer Woche gefunden wird und ein Molekulargewicht von etwa 68.000 und einen isoelektrischen Punkt von 5,4 bis 5,6 aufweist, wobei diese Hybridomas durch Verschmelzung von Myelomzellen mit Milzzellen von Tieren, die mit diesem Protein immunisiert worden sind, erhalten werden.

9. Hybridomas nach Anspruch 8, bei denen die biologischen Materialien von Mäusen herstammen.

10. Monoclonale Antikörper, die spezifisch für ein Protein sind, wie es in Anspruch 1 angegeben ist, das ein Molekulargewicht von etwa 68.000 und einen isoelektrischen Punkt von 5,4 bis 5,6 aufweist und spezifisch in biologischen Materialien von Embryonen/Föten oder Neugeborenen mit einem Lebensalter von bis zu einer Woche gefunden wird.

11. Monoclonale Antikörper nach Anspruch 10, die durch Hybridomas erzeugt werden, die durch Verschmelzung von Myelomzellen mit Milzzellen von Tieren erhalten werden, die mit einem Protein immunisiert worden sind, das ein Molekulargewicht von etwa 68.000 und einem isoelektrischen Punkt von 5,4 bis 5,6 hat und spezifisch in biologischen Materialien von Embryonen/Föten oder Neugeborenen mit einem Lebensalter von bis zu einer Woche gefunden wird.

12. Monoclonale Antikörper nach Anspruch 11, bei denen die biologischen Materialien von Mäusen herstammen.

13. Ein Verfahren zum Herstellen von monoclonalen Antikörpern gegen ein Protein, wie es in Anspruch 1 angegeben ist, das spezifisch in biologischen Materialien von Embryonen/Föten oder Neugeborenen mit einem Lebensalter von bis zu einer Woche gefunden wird und ein Molekulargewicht von etwa 68.000 und einen isoelektrischen Punkt von 5,4 bis 5,6 aufweist, wobei dieses Verfahren die Schritte umfaßt, daß (A) Myelomzellen mit Milzzellen von Tieren, die mit diesem Protein immunisiert worden sind, verschmolzen werden, um Hybridomas zu erhalten, und (B) unter diesen Hybridomas diejenigen ausgewählt werden, die in der Lage sind, monoclonale Antikörper gegen dieses Protein zu erzeugen und daß diese monoclonalen Antikörper unter Verwendung dieser Hybridomas erzeugt werden.

14. Ein Verfahren zum Herstellen von monoclonalen Antikörpern nach Anspruch 13, bei dem der Schritt (B) umfaßt, daß diese Hybridomas, die in der Lage sind, monoclonale Antikörper zu erzeugen, in einem Gewebekulturfluid gezüchtet werden, diese monoclonalen Antikörper in diesem Kulturfluid erzeugt und aufgesammelt werden und dann diese monoclonalen Antikörper von diesem Kulturfluid isoliert werden.

15. Ein Verfahren zum Herstellen von monoclonalen Antikörpern nach Anspruch 13, bei dem der Schritt (B) umfaßt, daß die Hybridomas, die in der Lage sind, monoclonale Antikörper zu erzeugen, in das Peritoneum einer nackten Ratte oder einer Ratte mit Immunsuppression transplantiert werden, um Aszites-Tumor zu induzieren, diese monoclonalen Antikörper in dem Aszites erzeugt und angesammelt werden und dann die monoclonalen Antikörper von dem Aszites isoliert werden.

16. Ein Verfahren zum Herstellen von monoclonalen Antikörpern nach den Ansprüchen 13, 14 oder 15, bei dem die biologischen Materialien von Mäusen herstammen.

17. Ein Verfahren zum Isolieren eines Proteins, das die Schritte umfaßt, daß ein Gemisch, das ein Protein enthält, wie es in Anspruch 1 angegeben ist, mit einem Träger, der Antikörper gegen das Protein, wie es in Anspruch 1 angegeben ist, trägt, in Kontaktgebracht wird und dieses Protein von den Bestandteilen, die auf dem Träger adsorbiert sind, selektiv eluiert und isoliert werden.

18. Ein Verfahren zum Isolieren eines Proteins nach Anspruch 17, bei dem diese Antikörper polyclonale Antikörper sind, wie sie in Anspruch 6 angegeben sind.

19. Ein Verfahren zum Isolieren eines Proteins nach Anspruch 17, bei dem diese Antikörper monoclonale Antikörper sind, wie sie in Anspruch 10 angegeben sind.

20. Eine pharmazeutische Zusammensetzung, die ein Protein enthält, wie es in Anspruch 1 angegeben ist.

21. Eine pharmazeutische Zusammensetzung, die Antikörper enthält, wie sie in Anspruch 6 angegeben sind.

22. Eine pharmazeutische Zusammensetzung, die Antikörper enthält, wie sie in Anspruch 10 angegeben sind.

## Revendications

1. Protéine que l'on trouve spécifiquement dans des substances biologiques provenant d'embryons/foetus ou de nouveaux-nés âgés d'une semaine au plus, et présentant un poids moléculaire d'environ 68 000 et un point isoélectrique de 5,4 à 5,6, ayant
a) une séquence amino-terminale d'aminoacides 
Leu-His-Glu-Asn-Glu-Phe-Gly-Ile-Ala-Ser-Thr-Leu-
Asp-Ser- ;
 et
b) les compositions en sucres et aminoacides suivantes :
| Composition en sucres (proportion molaire) : | |
|---|---|
| Galactose | environ 1 |
| Mannose | environ 1,42 |
| Fructose | environ 0,32 |
| Glucose | environ 0,07 |
| Glucosamine | environ 1,06 |
| Galactosamine | environ 0,10 |
| Acide sialique | environ 0,18 |
| Composition en aminoacides (proportion molaire) : | |
|---|---|
| Acide aspartique | environ 43,2 |
| Thréonine | environ 26,1 |
| Sérine | environ 35,8 |
| Acide glutamique | environ 58,4 |
| Glycine | environ 30,2 |
| Alanine | environ 30,6 |
| Cystéine | environ 6,9 |
| Valine | environ 23,3 |
| Méthionine | environ 5,2 |
| Isoleucine | environ 9,5 |
| Leucine | environ 48,4 |
| Tyrosine | environ 8,6 |
| Phénylalanine | environ 17,4 |
| Lysine | environ 26,3 |
| Ammonium | environ 90,1 |
| Histidine | environ 10,6 |
| Arginine | environ 18,2 |
| Proline | environ 26,5 |

2. Protéine selon la revendications 1, dans laquelle lesdites substances biologiques sont d'origine murine.

3. Protéine selon la revendication 1, dans laquelle ladite protéine est isolée par chromatographie d'affinité à partir d'un liquide extrait de tissus ou de cellules de souris, en utilisant une lectine comme agent d'affinité.

4. Procédé de préparation d'une protéine selon la revendication 1, comprenant les étapes consistant à amener le liquide extrait de tissus ou de cellules d'embryons/foetus ou de nouveaux-nés âgés d'une semaine au plus, en contact avec un support porteur d'une lectine, et à éluer sélectivement et isoler la protéine ayant un poids moléculaire d'environ 68 000 daltons et un point isoélectrique de 5,4 à 5,6, à partir des constituants adsorbés sur le support.

5. Procédé de préparation d'une protéine selon la revendication 4, caractérisé en ce que ledit extrait provient de tissus murins, ou de cellules d'embryons/foetus murins, ou de souris nouveaux-nées âgées d'une semaine au plus.

6. Anticorps polyclonaux, dirigés contre une protéine selon la revendication 1, qui présente un poids moléculaire d'environ 68 000 et un point isoélectrique de 5,4 à 5,6 et se trouve spécifiquement dans des substances biologiques provenant d'embryons/foetus ou nouveaux-nés âgés d'une semaine au plus, et obtenus par immunisation d'animaux avec ladite protéine.

7. Anticorps polyclonaux selon la revendication 6, dans lesquels lesdites substances biologiques sont d'origine murine.

8. Hybridomes capables de produire des anticorps monoclonaux spécifiques à une protéine selon la revendication 1, qui se trouve spécifiquement dans des substances biologiques provenant d'embryons/foetus ou de nouveaux-nés âgés d'une semaine au plus, et qui a un poids moléculaire d'environ 68 000 et un point isoélectrique de 5,4 à 5,6, lesdits hybridomes étant obtenus par fusion de cellules de myélome avec des cellules de rates provenant d'animaux immunisés avec ladite protéine.

9. Hybridomes selon la revendication 8, dans lesquels les substances biologiques sont d'origine murine.

10. Anticorps monoclonaux spécifiques à une protéine selon la revendication 1, qui présente un poids moléculaire d'environ 68 000 et un point isoélectrique de 5,4 à 5,6 et se trouve spécifiquement dans des substances biologiques provenant d'embryons/foetus ou de nouveaux-nés âgés d'une semaine au plus.

11. Anticorps monoclonaux selon la revendication 10, qui sont produits par des hybridomes obtenus par fusion de cellules de myélome avec des cellules de rates d'animaux immunisés avec une protéine ayant un poids moléculaire d'environ 68 000 et un point isoélectrique de 5,4 à 5,6 et qui se trouve spécifiquement dans des substances biologiques provenant d'embryons/foetus ou de nouveaux-nés âgés d'une semaine au plus.

12. Anticorps monoclonaux selon la revendication 11, dans lesquels les substances biologiques sont d'origine murine.

13. Procédé de préparation d'anticorps monoclonaux dirigés contre une protéine selon la revendication 1, qui se trouve spécifiquement dans des substances biologiques provenant d'embryons/foetus ou de nouveaux-nés âgés d'une semaine au plus et présente un poids moléculaire d'environ 68 000 et un point isoélectrique de 5,4 à 5,6, ledit procédé comprenant les étapes consistant à (A) fusionner des cellules de myélome avec des cellules de rates d'animaux immunisés avec ladite protéine, pour obtenir des hybridomes, et (B) parmi les hybridomes, sélectionner ceux capables de produire des anticorps monoclonaux dirigés contre ladite protéine, et à produire lesdits anticorps monoclonaux en utilisant lesdits hybridomes.

14. Procédé de préparation d'anticorps monoclonaux selon la revendication 13, dans lequel l'étape (B) consiste à cultiver lesdits hybridomes capables de produire lesdits anticorps monoclonaux dans un milieu de culture tissulaire, produire et accumuler lesdits anticorps monoclonaux dans ledit milieu de culture et ensuite, isoler lesdits anticorps monoclonaux à partir dudit milieu de culture.

15. Procédé de préparation d'anticorps monoclonaux selon la revendication 13, dans lequel l'étape (B) consiste à transplanter lesdits hybridomes capables de produire lesdits anticorps monoclonaux dans le péritoine d'un rat nude ou d'un rat à immunité réduite pour provoquer une tumeur ascitique, produire et accumuler lesdits anticorps monoclonaux dans le liquide ascitique et ensuite, isoler lesdits anticorps monoclonaux à partir dudit liquide ascitique.

16. Procédé de préparation d'anticorps monoclonaux selon les revendications 13, 14 et 15, dans lequel lesdites substances biologiques sont d'origine murine.

17. Procédé pour isoler une protéine, comprenant les étapes consistant à mettre un mélange, contenant une protéine selon la revendication 1, en contact avec un support porteur d'anticorps dirigés contre la protéine selon la revendication 1, et à éluer sélectivement et isoler ladite protéine à partir des constituants adsorbés sur le support.

18. Procédé pour isoler une protéine selon la revendication 17, dans lequel lesdits anticorps sont des anticorps polyclonaux selon la revendication 6.

19. Procédé pour isoler une protéine selon la revendication 17, dans lequel lesdits anticorps sont des anticorps monoclonaux selon la revendication 10.

20. Composition pharmaceutique contenant une protéine selon la revendication 1.

21. Composition pharmaceutique contenant des anticorps selon la revendication 6.

22. Composition pharmaceutique contenant des anticorps selon la revendication 10.
